# EUROPEAN PATENT APPLICATION

(11) **EP 2 015 074 A1**
(43) Date of publication of application: **14.01.2009**
(21) Application number: 07011633.0
(22) Date of filing: 14.06.2007
(51) Int. Cl.: G01N 33/68, G01N 33/543

(54) **Preparation of samples for LC-MS/MS using magnetic particles**

(71) Applicant: Roche Diagnostics GmbH, 68305 Mannheim (DE); F.HOFFMANN-LA ROCHE AG, 4070 Basel (CH); Ludwig-Maximilians-Universität, 80539 München (DE)
(72) Inventor: Kobold, Uwe, 82362 Weilheim (DE); Geiger, Albert, 82377 Penzberg (DE); Herrmann, Rupert, 82362 Weilheim (DE); Vogeser, Michael, 81375 München (DE)

(57) **Abstract**

The present invention provides a novel procedure of preparing samples for analysis by way of mass spectrometry, preferably LC-MS/MS. Accordingly, functionalized magnetic particles with a hydrophobic surface are used for extracting low molecular weight compounds from complex liquid biological samples such as plasma, serum, whole blood or hemolyzed blood. The method of the invention includes (a) contacting the sample with an amount of functionalized magnetic particles with a hydrophobic surface, (b) incubating the sample and the particles, thereby adsorbing the compound to the hydrophobic surface, (c) separating the particles by applying a magnetic field and removing the liquid, (d) optionally washing the particles, (e) eluting the compound from the particles.

## Description

The invention is directed to the field of sample preparation for liquid chromatography-tandem mass spectrometry. Particularly, the invention concerns preparation procedures suitable for the detection of low molecular weight substances in complex biological samples such as plasma, serum or whole blood.

### Background of the Invention

Liquid chromatography (LC) is an extremely important analytical technique which is used for the separation, identification and quantitation of an analyte of interest even if present in a complex mixture of different sample constituents. During LC the chemical components in a mixture are carried through a stationary phase by the flow of a liquid mobile phase. Separation in liquid chromatography is achieved by means of differences in the interactions of the analytes with both the mobile and stationary phases. As the skilled artisan appreciates both a stationary phase and a mobile phase appropriate to the analytes under investigation have to be chosen. In addition, the user will identify chromatographic conditions appropriate to maintain the sharpness of analyte bands as a sample moves through the stationary phase column to the detector.

High Performance Liquid Chromatography, also known as High Pressure Liquid Chromatography, abbreviated as HPLC, is a special form of liquid chromatography and nowadays used frequently in biochemistry and analytical chemistry. The analyte is forced through a column of the stationary phase in a liquid (mobile phase) at high pressure, which decreases the time the separated components remain on the stationary phase and thus the time they have to diffuse within the column. This leads to narrower peaks in the resulting chromatogram and thence to better resolution and sensitivity as compared to LC.

The mobile phase is chosen to ensure solubility of the sample solutes. For the stationary phase, preferably microparticulate silica (bare or chemically modified) is used, because its high surface area accentuates the differences in solute-stationary phase interactions. The use of a stationary phase that interacts strongly with solutes relative to solute mobile-phase interactions will result in very long retention times, a situation which is not analytically useful. Hence the stationary phase must be selected so as to provide weak to moderate solute interactions relative to those in the mobile phase. As a consequence, the nature of the solute governs the type of LC selected. The stronger interactions should occur in the mobile phase to ensure sample solubility and ready elution, while the stationary phase should be responsive to more subtle differences among the solutes. For example, polar neutral compounds are usually better analyzed using a polar mobile phase together with a nonpolar stationary phase that distinguishes subtle differences in the dispersive character of the solutes. One of the powerful aspects of HPLC is that the mobile phase can be varied to alter the retention mechanism. Modifiers can be added to the mobile phase to control retention. For example, pH is an important variable in aqueous mobile phases.

Five general classes of LC can be distinguished:
1. Normal-phase chromatography calls for the use of a polar stationary phase in conjunction with a non-polar (dispersive) mobile phase.
2. Reversed-phase chromatography, the opposite possibility, calls for the use of a non-polar stationary phase and a polar mobile phase (composed of one or more of the polar solvents, e.g. water, methanol, acetonitrile, and tetrahydrofuran).
3. Ion-exchange chromatography involves ionic interactions. In this case the mobile phase must support ionization to ensure solubility of ionic solutes. The stationary phase must also be partially ionic to promote some retention. Consequently, the interactions with the stationary phase are strong, and this is usually reflected in longer analysis times and broad peaks.
4. Size-Exclusion chromatography involves separations based on molecular size alone and ideally requires that there be no energetic interaction of the solutes with the stationary phase.
5. Affinity chromatography is based on a specific interaction, e.g. between the members of a specific binding pair, like antigen and corresponding antibody or receptor and corresponding ligand. For example a first partner of a binding pair is bound to an appropriate stationary phase and used to capture the second partner of the binding pair. The second partner can be released and isolated by appropriate means.

The general classification of separation principles given above is not exhaustive and therefore is non-limiting; there are other separation principles which can be used for the separation of liquid samples, e.g. hydrophobic interaction chromatography, hydrophilic interaction chromatography, ion-pair chromatography, and molecular imprinted materials based separation.

The analyte of interest can be detected by any appropriate means. Appropriate and preferred detectors sense the presence of a compound passing through, and provide an electronic signal to a recorder or computer data station. The output is usually in the form of a chromatogram and a substance of interest is usually found in a certain peak. The peak area or peak height can be used to quantify the amount of analyte present in the sample investigated.

The detector for an HPLC system is the component that emits a response due to the eluting sample compound and subsequently signals a peak on the chromatogram. It is positioned immediately posterior to the stationary phase in order to detect the compounds as they elute from the column. The bandwidth and height of the peaks may usually be adjusted using the coarse and fine tuning controls, and the detection and sensitivity parameters may also be controlled by the skilled artisan. There are many types of detectors that can be used with HPLC. Some of the more common detectors include: Refractive Index (RI), Ultra-Violet (UV), Fluorescent, Radiochemical, Electrochemical, Near-Infra Red (Near-IR), Mass Spectromety (MS), Nuclear Magnetic Resonance (NMR), and Light Scattering (LS).

Refractive Index (RI) detectors measure the ability of sample molecules to bend or refract light. This property for each molecule or compound is called its refractive index. For most RI detectors, light proceeds through a bi-modular flow-cell to a photodetector. One channel of the flow-cell directs the mobile phase passing through the column while the other directs only the mobile phase. Detection occurs when the light is bent due to samples eluting from the column, and this is read as a disparity between the two channels.

Fluorescent detectors measure the ability of a compound to absorb then re-emit light at given wavelengths. Each compound has a characteristic fluorescence. The excitation source passes through the flow cell to a photodetector while a monochromator measures the emission wavelengths.

Radiochemical detection involves the use of radiolabeled material, usually tritium (3H) or carbon-14 (14C). It operates by detection of fluorescence associated with beta-particle ionization, and it is most popular in metabolite research. Electrochemical detectors measure compounds that undergo oxidation or reduction reactions. This is usually accomplished by measuring gain or loss of electrons from migrating samples as they pass between electrodes at a given difference in electrical potential.

Mass spectrometry is an analytical technique used to measure the mass-to-charge ratio (m/z or m/q) of ions. It is most generally used to analyze the composition of a physical sample by generating a mass spectrum representing the masses of sample components. The technique has several applications, including: identifying unknown compounds by the mass of the compound and/or fragments thereof; determining the isotopic composition of one or more elements in a compound; determining the structure of compounds by observing the fragmentation of the compound; quantitating the amount of a compound in a sample using carefully designed methods (mass spectrometry is not inherently quantitative); studying the fundamentals of gas phase ion chemistry (the chemistry of ions and neutrals in vacuum); determining other physical, chemical or even biological properties of compounds with a variety of other approaches.

A mass spectrometer is a device used for mass spectrometry, and produces a mass spectrum of a sample to analyze its composition. This is normally achieved by ionizing the sample and separating ions of differing masses and recording their relative abundance by measuring intensities of ion flux. A typical mass spectrometer comprises three parts: an ion source, a mass analyzer, and a detector.

The kind of ion source is a contributing factor that strongly influences what types of samples can be analyzed by mass spectrometry. Electron ionization and chemical ionization are used for gases and vapors. In chemical ionization sources, the analyte is ionized by chemical ion-molecule reactions during collisions in the source. Two techniques often used with liquid and solid biological samples include electrospray ionization (ESI) and matrix-assisted laser desorption/ionization (MALDI). Other techniques include fast atom bombardment (FAB), thermospray, atmospheric pressure chemical ionization (APCI), secondary ion mass spectrometry (SIMS) and thermal ionisation.

Liquid-chromatography-tandem-mass spectrometry (LC-MS/MS) has been introduced in clinical chemistry (Vogeser M., Clin Chem Lab Med 41 (2003) 117-26). Advantages of this technology are high analytical specificity and accuracy, and the flexibility in the development of reliable analytical methods. In contrast to gas chromatography mass spectrometry (GC-MS) as the traditional mass spectrometic technology in clinical chemistry, LC-MS/MS has been shown to be a robust technology, allowing its application also in a large scale routine laboratory setting. Requirements for the preparation (clean-up) of sample material are limited compared to GC-MS, however, de-proteinizing is mandatory for small molecule target analyses.

Mere protein precipitation as presented by the state of the art may be sufficient for some LC-MS/MS methods, but in order to avoid ion-suppresion effects for very sensitive methods, more efficient extraction methods are usually required (Annesley, TM, Clin Chem (2003) 49:1041-4). "Off-line" or "on-line" solid phase extraction, or solvent extraction are the techniques currently used to solve this problem. Respective time consuming manual sample preparation protocols so far represent an important limitation for the large scale routine application of LC-MS/MS in the clinical laboratory. Therefore, automation of sample preparation for LC-MS/MS is a goal that is addressed by application of different technical principles:
Samples can be loaded into 96-well plates to be submitted to batch protein precipiation by centrifugation (Vogeser M and Spöhrer U, Clin Chem Lab Med (2006) 44:1126-30); this, however, is a discontinous process since plates have to be transferred into a centrifuge manually. Alternatively, filtration of precipitated samples using filtration plates and application of vacuum may be performed (Williams MG, et al., Biomed Chromatogr (2003) 17:215-8). Mere protein precipitation, however, does not allow analyte concentration.

Solid phase extraction with extraction plates or single extraction cardriges allows full automation with a continous work-flow from loading of samples until MS-analysis (Yang L, et al., J Chromatogr B Analyt Technol Biomed Life Sci (2004) 809:75-80; Alnouti Y, et al., J Chromatogr A (2005) 1080:99-106; Koal Tet al., Clin Chem Lab Med (2006) 44:299-305; Tarning J, et al., J Pharm Biomed Anal (2006) 41:213-8.). In these methods, too, vacuum or positive air pressure has to be applied during extraction which is technically demanding.

The use of magnetic particles has been most successfully introduced to the automation of heterogenous immunoassays years ago (Porstmann T and Kiessig ST, J Immunol Methods (1992) 150:5-21); respective particles used as the solid phase for extraction are ideally suited for automation, since this "solid phase" can be manipulated as a liquid. Today, this principle represents the predominant technology applied in a number automated immunoassay systems. Automated methods for DNA purification based on functionalized magnetic particles have been introduced to routine laboratories as well (Namvar L, et al., J Clin Microbiol (2005) 43:2058-64).

WO 2005/015216 and WO 2006/075185 disclose processes for the preparation of coated polymer particles containing superparamagnetic crystals. Porous, surface-functionalized particles are reacted with at least one polyisocyanate and at least one diol or at least one epoxide. WO 2005/015216 discloses that such beads are of utility in adsorption/desorption processes analogously to the mechanisms in (a) reversed phase chromatography or hydrophobic chromatography, and (b) hydrophobic interaction chromatography. Particularly, adsorption of a mixture of proteins to functionalized beads is described, followed by fractionation of the proteins by applying desorption buffers containing (a) increasing concentrations of acetonitrile and (b) decreasing concentrations of ammonium sulphate.

Extraction protocols for based on the use of magnetic particles have successfully been adapted for MALDI-TOF analyses. Zhang X, et al., J Biomol Tech (2004) 15:167-75 disclose the processing of human plasma samples using a magnetic bead-based hydrophobic interaction chromatography resin. Villanueva J, et al. Anal Chem (2004) 76:1560-70 disclose an automated sample preparation from human serum samples, wherein peptides are captured and concentrated using a reversed-phase batch processing and magnetic particles which are surface-derivatized with reversed-phase ligands. However, the document also discloses that in some cases, prior to reversed-phase extraction, sera were additionally subjected to incubation with additives such as urea, DTT or n-octylglucoside. Also, proteins were removed from sera by way of precipitation or filtration, or serum albumin was removed by affinity chromatography.

The problem to be solved by the present invention was to enhance sample preparation for quantitative target analysis of small molecule analytes with mass spectrometry means such as LC-MS/MS, whereby the analytes are extracted out of complex biological matrices like serum, plasma, whole blood or lysed whole blood.

The analysis of peptides prepared from serum or plasma using hydrophobic magnetic particles as described in the state of the art basically targets molecules with a molecular weight greater than 1,000 Da (Daltons) and up to about 20,000 Da. In whole blood, plasma and serum there is a plethora of compounds with a molecular weight below 1,000 Da. Among these, peptides are just one group out of many others, including amino acids, lipids and many different metabolites of the biochemical pathways. Thus, there exists a significant complexity of blood, plasma and serum samples with regards to small (i.e. <1,000 Da) molecular compunds.

The inventors unexpectedly found that the use of functionalized magnetic particles with a hydrophobic surface greatly enhances the process for extraction of small molecule analytes out of complex biological matrices such as whole blood, plasma, and serum. The magnetic particles according to the invention can reversibly bind low molecular weight compounds when the particles are added to the biological samples. A particular surprising finding was that the binding process is not disturbed when lipids, peptides and proteins are abundantly present in the sample matrix. This observation proved to be especially advantageous for extraction out of plasma, serum and whole blood in which these components, among others, are particularly abundant. After the binding step and by separating the particles with magnetic force from the remaining sample material, unbound components of the biological sample can be removed efficiently from the bound analytes. Elution of the analytes from the particles provides the desired low molecular weight compounds in a form which is sufficiently pure for analysis by mass spectrometry, such as LC-MS/MS analysis. Even more surprising, functionalized magnetic particles with a hydrophobic surface can be used to extract small molecule analytes out of whole blood samples which, prior to extraction, were subjected to hemolysis. Not only is the extraction (=sample preparation) procedure according to the invention suitable for qualitative detection of a desired analyte with a low molecular weight. Surprisingly, the use of functionalized magnetic particles with a hydrophobic surface also allows quantitative detection of the analyte using mass spectrometry as a detection means.

### Summary of the Invention

A first aspect of the present invention is the use of functionalized magnetic particles with a hydrophobic surface for extracting a compound with low molecular weight from a complex liquid biological sample. Another aspect of the invention is a method to purify a low molecular weight compound from a complex liquid biological sample, comprising the steps of (a) contacting the sample with an amount of functionalized magnetic particles with a hydrophobic surface, (b) incubating the sample and the particles, thereby adsorbing the compound to the hydrophobic surface, (c) separating the particles by applying a magnetic field and removing the liquid, (d) optionally washing the particles, (e) eluting the compound from the particles. A further aspect of the invention is a method to assay a compound with low molecular weight in a liquid biological sample by way of mass spectrometry, comprising the steps of (a) contacting the sample with an amount of functionalized magnetic particles with a hydrophobic surface, (b) incubating the sample and the particles, thereby adsorbing the compound to the surface, (c) separating the particles by applying a magnetic field and removing the liquid, (d) optionally washing the particles, (e) eluting the compound from the particles, and (f) detecting the compound in the eluate by way of mass spectrometry. A further aspect of the invention is a porous magnetic particle with two different surfaces, characterized in that the first surface engulfs the pore lumen and is functionalized with a chemical group selected from the group consisting of a C4-C30 alkyl group, a copolymer of 1-vinyl-pyrrolidon and a comdivinylbenzene, and a copolymer of styrene and divinylbenzene, and the second surface is the remaining surface around the pores, whereby the second surface is either determined by the chemical composition of the solid phase or functionalized with a chemical group which is different from the chemical group on the first surface.

### Detailed Description of the Invention

Certain terms are used with particular meaning, or are defined for the first time, in this description of the present invention. For the purposes of the present invention, the following terms are defined by their art-accepted definitions, when such exist, except that when those definitions conflict or partially conflict with the definitions set forth below. In the event of a conflict in definition, the meaning of the terms are first defined by the definitions set forth below.

The term "comprising" is used in the description of the invention and in the claims to mean "including, but not necessarily limited to".

The articles "a" and "an" are used herein to refer to one or to more than one (i.e. to at least one) of the grammatical object of the article. By way of example, "a compound" means one compound or more than one compound.

When designating a range of numerical values such as a concentration range, the range is indicated by the word "between", followed by a first value n1 and a second value n2. The lower boundary of the designated range is understood as being the value equal to or higher than the first value. The higher boundary of the designated range is understood as being the value equal to or lower than the second value". Thus, a value x the designated range is given by n1 ≤ x ≤ n2.

Further, it is understood that the term "about" in combination with a numerical value n indicates a value x in the interval given by the numerical value ±5% of the value, i.e. n - 0.05 * n ≤ x ≤ n + 0.05 * n. In case the term "about" in combination with a numerical value n describes a preferred embodiment of the invention, the value of n is most preferred, if not indicated otherwise.

The present invention deals with enhancements of sample preparation prior to the detection of analytes which are low molecular weight (= LMW) compounds. One or more LMW compounds can be present in the sample to be analyzed. A "low molecular weight compound" "small molecule", or a "molecule", "substance" or "compound" of "low molecular weight" is a molecule which exhibits a molecular weight of less than 5,000 Da (Da = Daltons), preferably less than 2,000 Da, more preferably less than 1,000 Da, most preferably less than 500 Da.

A compound or a composition is a "liquid" if at room temperature and normal atmospheric pressure the compound is in the "liquid" state and forms a liquid phase.

The terms "aqueous", "aqueous" phase and "aqueous" solution describe a liquid phase of which the solvent portion comprises water. However, other solvents such as a water-miscible organic cosolvent can be present in the solvent portion, too. In view of the presence of a cosolvent a solution is considered "aqueous" when between 30% and 100%, measured as volume by volume [volume by volume], of the solvent portion is water.

The term "sample" (or "sample material") as used herein refers to a mixture of compounds in which the analyte to be determined can be present and from which it can be purified, if present. The skilled person will find the invention to be particularly advantageous when the sample is a complex sample. A complex sample contains a plurality of organic and inorganic compounds which are desired to be separated from the analyte to be targeted, when the analyte is present in the sample. Usually, the sample is a biological sample.. The sample preferably may comprise any body fluid. Preferred test samples include blood, blood serum (= serum), blood plasma (= plasma), urine, saliva, sputum, cerebral fluid or synovial fluid. Other samples are samples of in vitro cell culture constituents, homogenates of bacteria, animal, plant or fungal cells or tissue, and cleared supernatants thereof.

Very much preferred samples are whole blood, serum, and plasma, with plasma or serum being most preferred. The blood plasma is preferably EDTA, heparin or citrate blood plasma.

According to the invention, a preferred biological sample is a liquid sample which, however, may contain cellular or other particulate material, e.g., whole blood. Other preferred liquid samples are serum and plasma.

As the skilled artisan will appreciate, any assay using material from the sample is made in vitro. The patient sample is discarded or stored afterwards. The patient sample is solely used for the in vitro method of the invention and the material of the patient sample is not transferred back into the patient's body.

Furthermore, a lysate of biological material or a cleared supernatant thereof is encompassed by the term "sample". A "lysate" can be obtained from sample such as material comprising tissue, cells, bacteria or viruses, whereby the structural integrity of the material is disrupted, e.g. by adding an organic solvent and/or a detergent and/or a chaotropic salt, or by applying an osmotic shock. The term "lysate" does not encompass treatment with a hydrolase enzyme such as a protease or a nuclease.

The term "solid phase" is understood as being a substrate which is insoluble in the aqueous and non-aqueous liquids with which the present invention is practiced.

The term "magnetic particle" denotes a particle with paramagnetic or superparamagnetic properties. That is to say, the particle is magnetically displaceable but does not retain any magnetisation in the absence of an externally applied magnetic field. Examples for magnetic particles and methods for producing them can be found in WO 2005/015216 as well as in the references cited in this document. Particularly preferred is a magnetic polymer particle which contains paramagnetic or superparamagnetic crystals. The surface of a magnetic polymer particle can be modified chemically.

The terms "functionalize" and "functionalization" as used herein relate to chemical modification of a solid phase (substrate) to provide a plurality of functional groups on the substrate surface. By a "functionalized surface" as used herein is meant a substrate surface that has been modified so that a plurality of functional groups are present thereon. The chemical modification of the solid phase comprises one or more chemical (functional) groups which are covalently linked to the solid phase or integral part of the solid phase. The functional groups are capable of mediating a physical interaction of an analyte with the solid phase, such that the analyte is bound non-covalently and reversibly. An example for a functional group is a hydrophobic group.

The term "biologically active compound" denotes a synthetic or non-synthetic substance which produces an effect on living matter or alters a function of living matter when contacted therewith. The term excludes compounds which are nutrients. Biological activity is usually dosage-dependent. A prominent kind of biological activity is a substance's toxicity.

The commonly accepted meaning of "LC-MS/MS" is liquid chromatography-tandem mass spectrometry. For LC-MS/MS liquid chromatographic separation, such as by high pressure liquid chromatography (HPLC) or another LC system, is combined on-line with mass spectrometry (MS) to result in enhanced sensitivity and specificity. In LC-MS/MS, the effluent from an HPLC is directed towards the inlet to the MS. Mass spectrometers can be of different types like quadrupol instruments (single quadrupol or triple quadrupol), time of flight instruments, ion trap instruments or hybrid instruments (example quadrupol - time of flight). Compounds eluting from the LC system are analyzed in real time as they elute. Two types of scans can be performed. One type measures the masses of the intact species eluting from the LC system. This can be done by scanning over a broad mass range (example 100 to 2,000 m/z) or by scanning one, two or several individual masses of interrest (selected ion monitoring = SIM). A second type of measurement is using gas phase fragmentation. Fragmentation can be performed in the ion source (e.g. electrospray, ESI or atmospheric pressure chemical ionisation, APCI), this is known as "in-source fragmentation". Fragmentation can be preferably performed after isolation of one or more specific precursor ions in a tandem MS. After fragmentation specific fragment ions are measured. This type of measurement is known as multiple reaction monitoring = MRM or selected reaction monitoring = SRM. MRM type of measurement ensures the highest sensitivity and specificity.

An exemplary technical problem underlying the present invention arises when complex samples containing an abundance of proteins are to be prepared for an assay by LC-MS/MS for LMW compounds as analytes. Conventionally, single samples a simple protein precipitation is performed. While the precipitation removes unwanted components from the liquid phase, the desired analyte remains in solution. However, the precipitation step necessitates a subsequent centrifugation step, or an equivalent means to separate the precipitate from the liquid phase (supernatant), before the latter can be processed and analyzed further. Particularly a centrifugation step results in a discontinous process. Having full automation of the sample preparation workflow in mind, the inventors set out to develop alternative and more advantageous solutions.

The inventors discovered that functionalized magnetic particles provide unexpected benefits in the workflow of sample preparation preceding the analysis of a LMW compound from complex liquid samples. Thus, a first aspect of the present invention is the use of functionalized magnetic particles with a hydrophobic surface for extracting a compound with low molecular weight from a complex liquid biological sample. In a preferred embodiment of the invention, the functionalized surface comprises one or more hydrophobic groups.

With great advantage, the method of the invention results in reduced ion suppression which has a positive impact on the quality of mass spectrometric analysis, e.g. by LC-MS/MS. In addition, the workflow using functionalized magnetic particles according to the invention leads to an increased purity of the analyte from the sample material. As a consequence, HPLC separation is less demanding with respect to separation efficiency, thereby reducing costs and inceasing speed of the analytical process. At the same time, the lifetime of the analytical HPLC columns is increased.

Very much preferred, the functionalized magnetic particles with a hydrophobic surface are used for the extraction of a compound with a molecular weight between 50 Da and 1,000 Da. More preferred, the molecular weight of the compound is between 50 Da and 800 Da, Even more preferred the molecular weight of the compound is equal to or less than 600 Da.

The technical effects of the present invention are also of general importance with respect to the goal of fully automated LC-MS/MS methods, since the inventors demonstrate for the first time the applicability of a magnetic particle based extraction protocol to small analyte quantitative LC-MS/MS target analysis. The particle technology offers substantial advantages compared to solid phase extration protocols which involve "immobile" extraction materials: minimized handling of solid consumables (cartridges, plates); minimized volumes of liquid extraction solutions; and in particular no technically demanding application of vacuum or pressure to the extraction materials.

Another aspect of the invention is a method to purify a low molecular weight compound from a complex liquid biological sample, comprising the steps of (a) contacting the sample with an amount of functionalized magnetic particles with a hydrophobic surface, (b) incubating the sample and the particles, thereby adsorbing the compound to the hydrophobic surface, (c) separating the particles by applying a magnetic field and removing the liquid, (d) optionally washing the particles, (e) eluting the compound from the particles. The procedure of step (c), that is separating the magnetic particles from the remaining liquid phase is well known to the skilled person. E.g., by way of applying the magnetic field, the particles can attracted to the wall of the container which holds the liquid sample and the magnetic particles. The liquid phase can then be aspirated, thereby separating the particles with any material adsorbed thereto from the remaining components of the sample. Subsequently, the particles can be washed with a washing buffer. The composition of the washing buffer is to be chosen such that the desired analyte remains bound to the particles.

Suitable and preferred particles to practice the present invention are magnetic nanoparticles and polymer particles containing magnetic pigment particles. However, other particles are possible. A further preferred particle is a porous particle which provides more than one functionalized surface and which provides restricted access to one particular functionalized surface. Very much preferred, the particles have a functionalized surface with one or more hydrophobic group to result in a hydrophobic surface area.

Magnetic particles (nanoparticles) with a hydrophobic surface can be generated by chemically coupling hydrophobic groups to Fe₃O₄, gamma-Fe₂O₃, mixed oxides of Fe₂O₃ and oxides of bivalent or trivalent metal ions, or mixtures thereof. To this end, procedures are disclosed in WO 00/26927 and elsewhere. In an alternative approach, polymer particles which contain paramagnetic or superparamagnetic crystals, can be prepared as described in WO 2005/015216 and WO 2006/075185. In these cases the modification chemistry targets the polymer. Styrenedivinylbenzene copolymers can be activatd e.g. using 1,4-butanediol diglycidylether and glycidol. Remaining epoxy functional groups can be modified using e.g. alkylalcohols. Alternatively, commercially available epoxy modified magnetic styrenedivinylbenzen copolymers (e.g. Dynabead M270 epoxy, INVITROGEN Corporation, 1600 Faraday Ave, Carlsbad, CA 92008,catalogue no. 143-01) can be used.

Very much preferred, the hydrophobic group with which the surface or part of the surface of the solid phase is functionalized is selected from the group consisting of an aliphatic linear, branched or cyclic saturated or unsaturated hydrocarbon residue, and an aromatic organic residue, whereby the aromatic residue may also comprise a heterocyclic residue.

According to the invention, the surface of the magnetic particles is even more preferably functionalized with C4-C30 alkyl residues, more preferred C8-C25, even more preferred C18. Other preferred hydrophobic particles are for example copolymers of 1-vinyl-pyrrolidone and divinylbenzene, and styrene and divinylbenzene, respectively.

The solid phase of the magnetic particles can be a porous or non-porous solid phase. A preferred porous solid phase has an average pore size of about 50 to 1000 nm, more preferred about 50 to 300 nm, even more preferred about 200 nm. Most preferred, the solid phase is functionalized exclusively on the surface surrounded by the pore lumen. Using such a porous solid phase, also known as "restricted access material", the simultaneous function of a molecular sieve can be achieved, in addition to the interaction between the analyte and the functionalized surface. That is to say, higher molecular compounds are excluded from access to the pore lumen due to steric hinderance, and therefore from interacting with the functionalized surface of the solid phase.

The procedure according to the invention is particularly advantageous for the separation of a biologically active LMW compound from a liquid biological sample.

Preferably, the liquid sample is selected from the group consisting of whole blood, serum, and plasma. In addition and more preferred, the liquid sample can be hemolyzed blood, that is to say a blood sample in which the integrity of cellular constituents has been disrupted. E.g., erythrocytes in a blood simple can be lysed by applying an osmotic shock.

When using magnetic particles with a hydrophobic surface a preferred LMW compound has some lipophilic character and is capable of interacting directly with the hydrophobic surface. Alternatively, the LMW compound is capable of participating in hydrophobic interactions upon addition of ion pairing reagents.

In this context, lipophilic character means, the LMW compound shows a high binding affinity to the hydrophobic surface of the magnetic beads in an aqueous solution with high water content and this binding can be released by lowering the water content and increasing the organic content of the solution.

By way of example, a water content of between 100% and 90% is considered to be an aqueous solution with a high water content. Increasing in this solution the concentration of a water-miscible organic solvent such as methanol from 10%to 80% results in an aqueous solution with a low water content.

In this context, other organic solvents could be acetonitrile, ethanol or other aliphatic alcohols or other water-miscible organic compounds like dimethylformamide (DMF) or dimethylsulfoxide (DMSO). Ion pairing reagents could be for example trifluoroacetic acid, ammonium salts or sulfonium salts.

Particularly, the invention is practiced to purify biologically active LMW compounds, e.g. from complex liquid samples such as body fluids. Very much preferred biologically active LMW compounds are organic compounds which are soluble in matrices with high water content, e.g. in whole blood or serum, and which are also soluble in mixtures of water and organic solvents.

Solubility of a LMW compound can be influenced by the pH of the solution if protonation and deprotonation of the LMW compound is possible.

Typical LMW compounds for which the invention is very much suited are pharmaceutical compounds. There is particular utility for immunosuppressiva such as Rapamycin, Tacrolimus, MPA, Everolimus, Sirolimus, and metabolites thereof. Other very much preferred LMV compounds are endogeneous metabolites such as Folic acid, vitamines and hormones.

A further preferred target analyte according to the present invention is a drug of abuse. A drug of abuse is preferably selected from the group consisting of amphetamine, cocaine and cocaine metabolites like benzoylecgnonine, methamphetamine, opiate and opiate derivatives, cannabinoids like tetrahydrocannabinol, and phencyclidine. A further preferred target analyte is folate especially the total folate as comprised in both the blood plasma and in the red blood cells. Well-known immunosuppressive drugs are e.g. mycophenolate mofetil (MMF), rapamycin (RAPA also known as sirolimus) and tacrolimus (FK-506). Therapeutic drug monitoring for immunosuppressive drugs is especially important for transplant patients as well as for patients suffering from AIDS (cf. e.g.: Drug Ther Perspect 17(22) (2001) 8-12). Most patients who undergo solid organ transplantation require lifelong immunosuppressive therapy to prevent allograft rejection. Because many immunosuppressive agents have narrow therapeutic ranges and are associated with various toxicities and the potential for drug interactions, the use of therapeutic drug monitoring (TDM) in conjunction with clinical assessment of patients is particularly important.

Therefore, an immunosuppressive drug as a preferred analyte is selected from the group consisting of cyclosporine (CsA), mycophenolate mofetil (MMF), rapamycin (RAPA also known as sirolimus), tacrolimus (FK-506) azathioprine (AZA), and methylprednisolone (MP).

Also preferred is a target analyte that is present in a red blood cell. Preferred analytes to be measured from a differentially hemolyzed whole blood sample are sirolimus, tacrolimus and folate.

Mycophenolate mofetil is a prodrug. After oral administration, mycophenolate mofetil (MMF) undergoes rapid hydrolysis in the intestine and blood to form its active metabolite mycophenolic acid (MPA). MMF is widely available and is approved in the US and UK for the prevention of renal, hepatic or cardiac allograft rejection in combination with corticosteroids and cyclosporin. The drug has demonstrated superiority over azathioprine in reducing the incidence of acute rejection of renal allografts. The therapeutic trough concentration is in the range of 1-3.5 mg/l. MMF can be measured from plasma and from whole blood.

Tacrolimus is a macrolide antibiotic that was first approved by the US Food and Drug Administration (FDA) in 1994 for the prevention of liver allograft rejection. It is up to 100 times more potent than cyclosporin *in vitro,* and clinically, it is associated with a greater reduction in the incidence of tissue rejection. Tacrolimus has demonstrated efficacy both as primary immunosuppressive therapy in patients undergoing various transplantation procedures, and as rescue therapy for patients with refractory acute allograft rejection after liver or kidney transplantation. The therapeutic trough concentration is in the range of 5-20 µg/l.

Since at least part of the tacrolimus present in the circulation is compartmented within erythrocytes, a whole blood sample is used in the clinical routine measurement of this drug. Tacrolimus can e.g. be detected by high performance liquid chromatography (HPLC), HPLC mass spectrometry (MS), radio receptor assay (RRA), or by an immunoassay (IA). The latter two methodologies do not detect tacrolimus and certain of its various metabolites with the same sensitivity. This may lead to an interference in the procedure used (Murthy, J. N., et al., Clin. Biochem. 31/8 (1998) 613-617). At least in the detection of the various tacrolimus metabolites the HPLC-MS-procedure may be considered the gold standard. All the procedures mentioned above, however, require the extraction of tacrolimus from whole blood. Usually acetonitrile is used in clinical routine for the extraction of tacrolimus from whole blood and no method appears to exist that would allow for an online measurement of tacrolimus from a whole blood sample.

Sirolimus is, like tacrolimus, a macrolide antibiotic. It was first approved in 1999 by the US FDA for the prevention of allograft rejection after kidney transplantation, and indeed has shown promising results in this respect when used acutely in combination with cyclosporin and corticosteroids. In vitro, sirolimus is up to 100 times more potent than cyclosporin, and clinically, it may exhibit synergism with cyclosporin, perhaps permitting a reduction in cyclosporin dosage. The therapeutic trough concentration is in the range of 5-15 µg/l.

As for tacrolimus, a significant amount of sirolimus is present within erythrocytes. Therefore extraction of a whole blood sample is required no matter which detection method is used. In clinical routine a sample suspected to comprise sirolimus is subjected to HPLC and sirolimus is detected by ultraviolet light (UV) or by MS/MS. Recently also a microparticle enzyme immunoassay has been described (Jones, K., et al., Clinical Therapeutics 22/suppl. B (2000) B49-B61).

Folate is the collective name of a group of related molecules differing in oxidation state. Folates are part of the water-soluble vitamin B group and are important as coenzymes for homocysteine metabolism and in the transfer of one-carbon groups required for DNA replication. Inadequate folate status is related to increased risk of neural tube defects, is associated with cardiovascular disease, anemia, with certain cancers and with Alzheimer's disease. Serum or plasma folate concentrations reflect recent dietary intake, whereas erythrocyte folate concentrations are more indicative of body stores (E.W. Gunter et al, Clin Chem. 42, 1996, 1689-1694; Z. Fazili et al Clin Chem. 51, 2005, 2318-2325; C.M. Pfeiffer et al Clin Chem 50, 2004, 423-432). Erythrocyte total folate (red blood cell folate = RBC-folate) is the best measure of whole body folate status. Recent studies have shown that 5-methyl tetrahydrofolate is the dominant folate vitamer in circulating erythrocytes. For the diagnosis of folate deficiency it is recommended that determinations are performed not only from serum or from plasma but also from erythrocytes, since folate is localized to more than 95% in the latter. The concentration in the erythrocytes more truly reflects the actual folate status.

A further surprising finding was that the purification method according to the invention, when used for sample preparation, provides a LMW compound in a suitable form for analysis by means of liquid chromatography and mass spectrometry. Another aspect of the invention is therefore a method to assay a compound with low molecular weight in a liquid biological sample by way of mass spectrometry, comprising the steps of (a) contacting the sample with an amount of functionalized magnetic particles with a hydrophobic surface, (b) incubating the sample and the particles, thereby adsorbing the compound to the surface, (c) separating the particles by applying a magnetic field and removing the liquid, (d) optionally washing the particles, (e) eluting the compound from the particles, and (f) detecting the compound in the eluate by way of mass spectrometry.

In step (f) the compound is detected by LC/MS. A more preferred way of analyzing the eluate, however, is achieved using liquid chromatography-tandem mass spectrometry (LC-MS/MS).

The sample preparation method described here was found to result in very pure extracts obtained from human sample materials. As an effect, analytical interferences are reduced when applying liquid chromatography-tandem mass spectrometry. Ion suppression represents the most relevant analytical interference of liquid chromatography-mass spectrometry (LC-MS). Ion suppression results from the presence of an excess of non-target analyte matrix molecules in the ion source during ionization. Ion suppression effects compromise with lower concentration limits of quantification in LC-MS methods and therefore limit the applicability of LC-MS in the field of in-vitro diagnostics in general. Ion suppresion can have differential impact on the ionization yield obtained for an target analyte and its individual internal standard compound, respectively. Since the severity of ion suppression may differ substantially between samples (in particular between calibration samples and patients' samples) inaccuracy can arise from ion suppression.

An essential advantage of the sample preparation technique described herein is the option of full automation of all steps included in the extraction process. In particular when combined with the technique of liquid-chromatography tandem-mass spetrometry (LC-MS/MS) this enables the development of dedicated analytical instrument for use in routine clinical laboratories. Introduction of unprocessed samples to an instrument today are a prerequisite for the application of analytical platforms in the clinical laboratory. This can be accieved with the sample preparation technique described here. A further prerequisite is a high throughput capacity of the analytical platform with short analytical run times and highest analytical specifity. Respective features are best realized by LC-MS/MS, in particular when compared with single stage LC-MS and MALDI-TOF techniques.

In a preferred embodiment the detecting of an analyte using a method according to the present invention is performed by mass spectrometry. Compared with other detection techniques mass spectrometry gives highest specificity especially if combined with tandem mass spectrometry. With tandem mass spectrometry detection is a two dimensinal process

The following examples and figures are provided to aid the understanding of the present invention, the true scope of which is set forth in the appended claims. It is understood that modifications can be made in the procedures set forth without departing from the spirit of the invention.

### Description of the Figures

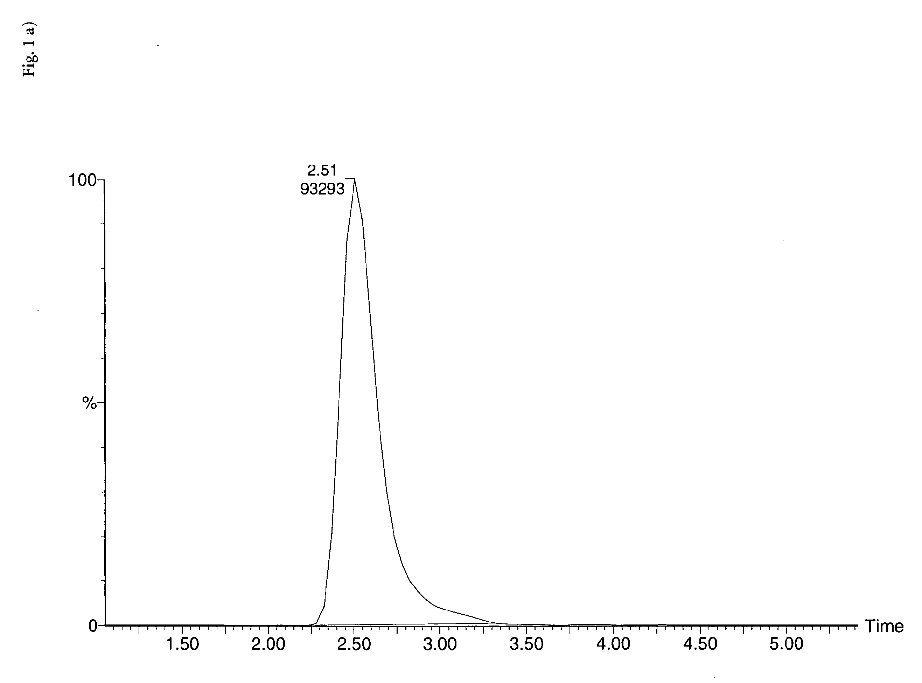

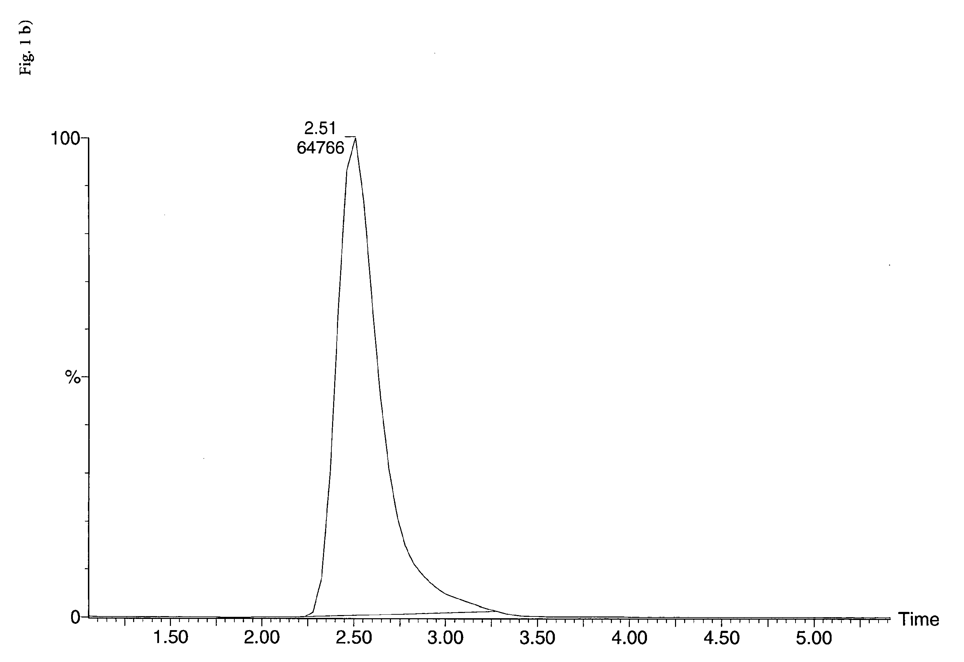

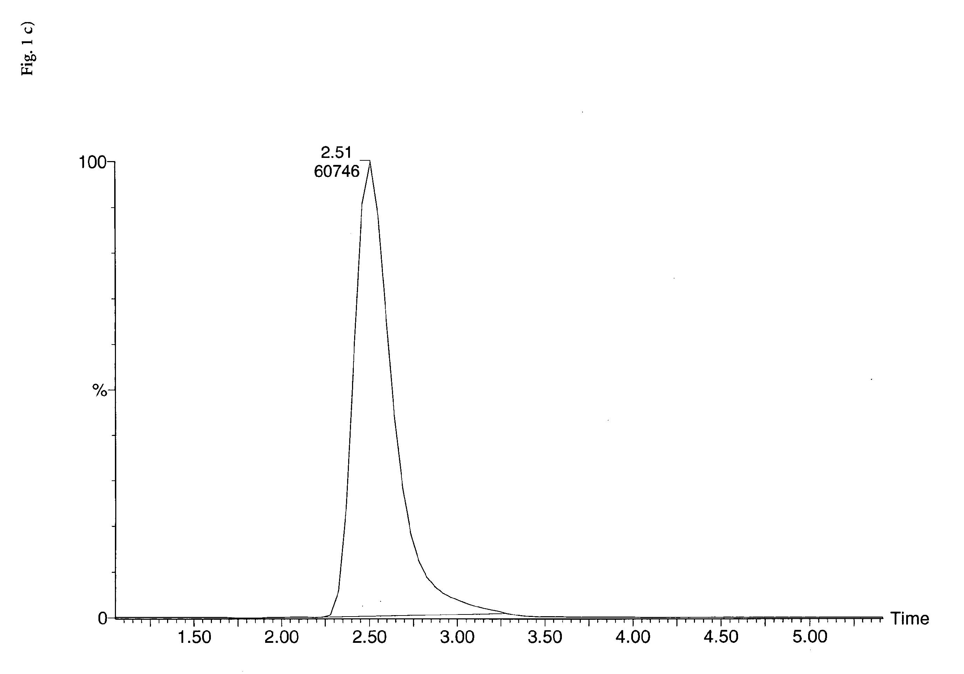

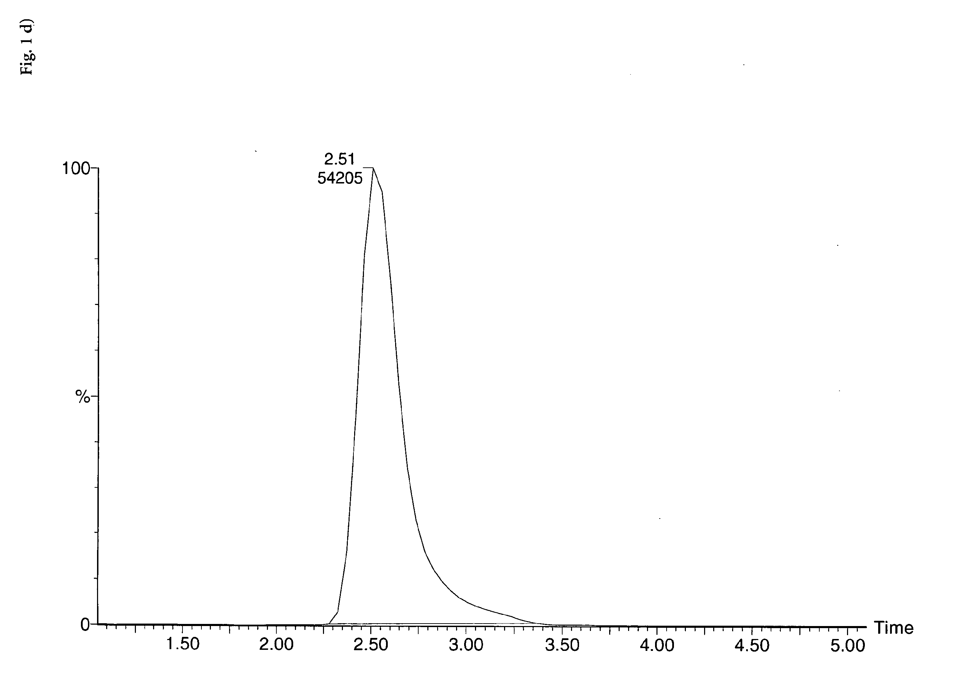

- Figure 1 a) - d):: Representative LC-MS/MS multiple reaction monitoring chromatograms obtained from different samples submitted to sample preparation
a) injection of pure solution of itraconazole, undiluted
b) injection of extract obtained from a pure solution of itraconazole (initial concentration 1.000 µg/l; tenfold dilution during extraction process) c) injection of extract obtained from a serum sample spiked with itraconazole (initial concentration 1.000 µg/l; tenfold dilution during extraction process)
d) injection of material obtained by simple protein precipitation (state-of-the-art) with acetonitrile from a serum sample spiked with itraconazole (initial concentration 1.000 µg/l; tenfold dilution during extraction process)

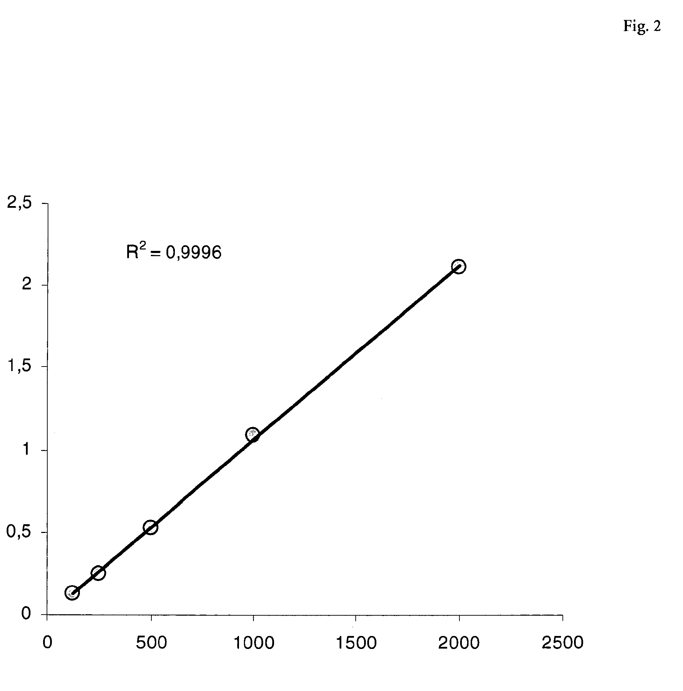

- **Figure 2:**: Calibration line for measurement of itracoanzole obtained from calibration materials submitted to sample preparation by microparticles. Ordinate: Response LC-MS/MS; peak area itraconazole internal standard. Abscissa: Concentration in µg/l itraconazole.
- **Figure 3 a) - c):**: Representative LC-MS/MS multiple reaction monitoring chromatograms obtained from different patient whole blood samples submitted to sample preparation
a) containing Tacrolimus, representative results for
   a.1 ) 2.1 µg/l,
   a.2) 10.5 µg/l, and
   a.3) 39.7 µg/l
b) containing Sirolimus
   b.1) 2.1 µg/l,
   b.2) 10.5 µg/l, and
   b.3) 47.2 µg/l
c) containg Everolimus
   c.1) 2.1 µg/l,
   c.2) 11.7 µg/l, and
   c.3) 43.9 µg/l
- **Figure 4:**: Representative LC-MS/MS multiple reaction monitoring chromatograms obtained from different patient serum samples submitted to sample preparation containg Mycophenolic acid. A standard; B patient sample.

### Example 1

### Analysis of itraconazole from serum

For the demonstration of the invention the antimycotic drug itraconazole (CAS Registry/EC Number (RN) 84625-61-6) is used as a first representative small molecule target analyte.

In an exemplary case commercially available C18-functionalized magnetic particles (= beads) (Dynal^{®} Dynabeads^{®} RPC18 (Prod. No. 102.01), Invitrogen, Karlsruhe, Germany; 50 mg/ml, mean particle diameter 1 µm) are used. The beads have a surface modified with C18 alkyl groups. Such particles are so far specified for the preparation of serum samples for MS-based protein analyses.

Serum samples from human patients are extracted using magnetic particles. In general the standard handling protocol given by the manufacturer is followed. 15 µl of the resuspended particles are transferred into 1.5 ml standard polypropylene recation tubes placed in a magnetic particle separator (Dynal MPC-S Magnetic Particle Concentrator, Product No. 120.20, Invitrogen). A permanent magnet is introduced into the separator device, leading to immobilization of the beads on the wall of the tubes. The particle free liquid is removed with a pipette; then, the magnet is removed from the separator device.

For condtioning of the beads, 250 µl of 0.1% [volume by volume] trichloroacetic acid is dispensed into the tubes; the bead pellets are re-dissolved by multiple pipetting the liquid within the tubes. The magnet is applied again and trichloroacetic acid is removed. This conditioning step is repeated. The conditioned beads are resuspended in 50 µl of formic acid (0.1% [volume by volume]); 15 µl of the internal standard solution (R51012 (Vogeser M, et al. Clin Chem Lab Med (2003) 41:915-20), 1.000 µg/l in methanol / water (1/1 volume parts)) and 15 µl of serum are added. With a pipette the content of the tubes is aspirated and blown out several times for mixing. Adsorption of the analytes to the beads is allowed for two minutes. The sample matrix is removed with a pipette after again applying the magnet. Beads are washed two times with each 250 µl of water. After the second washing step the beads are resuspended in 75 µl of acetonitrile and 75 µl of formic acid (0.1% [volume by volume]) for analyte elution. After two minutes, the beads are captured magnetically and the supernatants are transferred into HPLC vials. The entire extraction procedure is performed at ambient temperature. LC-MS/MS analysis of the extract is performed as described previously (Vogeser M, et al. Clin Chem Lab Med (2003) 41:915-20) here using a Waters Micromass Quattro Ultima Pt instrument.

### Example 2

### Recovery rate of the extraction procedure for itraconazole and ion suppression properties

To investigate the recovery rate of the extraction procedure and to characterize ion suppression properties of the entire analytical method, pure solutions of itraconazole (1.000 µg/l and 100 µg/l, respecitvely, in methanol / water (10/90 [volume by volume]), and a serum sample spiked with itraconazole to a concentration of 1.000 µg/l are used. Injection of 7 µl of the pure solution with a concentration of 100 µg/l resulted in a peak area in the MRM trace of itraconazole of 80.978 (mean of three injections). The pure solution with a concentration of 1.000 µl is submitted to particle based extraction and 7 µl of the extract are injected. A mean peak area of 67.765 is observed. Since the extraction protocol includes a sample dilution by a factor of 10, these results correspond to an extraction recovery rate of 84%. The itraconazole spiked serum sample is extraced in the same way, and a mean peak area of 61.124 is observed, which is 9% less than found for the pure solution with the identical analyte concentration. This slightly lower signal may result from a lower recovery rate of the extration method when applied to serum or to an ion suppression effect caused by residual serum matrix, or from a combination of both. For comparison, the spiked serum sample is precipitated with acetonitrile (100 µl sample, 900 µl acetonitrile); a mean peak area of 49.095 is observed. Thus the overall extraction efficacy (including recovery rate and ion suppression effects) of the bead based protocol is found superior compared to protein precipitation.

### Example 3

### Validation with samples from itraconazole-treated patients

A validation protocol is performed in three independent analytical series. In each series a five-point calibration is performed using a commerically available calibration material for itraconazole measurement (2,000 µg/l, 1,000 µg/l, 500 µg/l, 250 µg/l, and 125 µg/l; Recipe, Munich, Germany). Aliquots of pooled serum from itraconazole treated patients is analyzed in triplicate in each series to calculate a total coefficient of variation (n=9). Commercially available quality control materials (ClinCheck, Recipe, Munich, Germany) in two concentration ranges (target concentrations 150 µg/l, and 2,900 µg/l) are analyzed in the three series.

Linear calibration lines (r>0.99) are obtained in all series. For the pooled serum a total coefficient of variation of 3.4% is observed (mean concentration 380 µg/l); all quality control samples are found within the predefined accepted ranges (±10% of the target concentrations).

### Example 4

### Analysis of blood samples containing Immunsupressiva

For the demonstration of the invention patient whole blood samples from patients treated with immunosupressent drugs are used as analytes. Immunosupressant drugs analysed are Tacrolimus, Sirolimus and Everolimus.

In an exemplary case commercially available C18-functionalized magnetic particles (= beads) (Dynal^{®} Dynabeads^{®} RPC18 (Prod. No. 102.01), Invitrogen, Karlsruhe, Germany; 50 mg/ml, mean particle diameter 1 µm) are used. The beads have a surface modified with C18 alkyl groups. Such particles are so far specified for the preparation of serum samples for MS-based protein analyses.

Whole blood samples from human patients are extracted using magnetic particles. In general the standard handling protocol given by the manufacturer is followed. 50 µl of the resuspended particles are transferred into 1.5 ml standard polypropylene recation tubes placed in a magnetic particle separator (Dynal MPC-S Magnetic Particle Concentrator, Product No. 120.20, Invitrogen). A permanent magnet is introduced into the separator device, leading to immobilization of the beads on the wall of the tubes. The particle free liquid is removed with a pipette; then, the magnet is removed from the separator device.

For condtioning of the beads, 250 µl of 0.1% [volume by volume] trichloroacetic acid is dispensed into the tubes; the bead pellets are re-dissolved by multiple pipetting the liquid within the tubes. The magnet is applied again and trichloroacetic acid is removed. This conditioning step is repeated. The conditioned beads are resuspended in 50 µl of formic acid (0.1% [volume by volume]); 50 µl of the internal standard solution and 50 µl of whole blood sample are added. With a pipette the content of the tubes is aspirated and blown out several times for mixing. Adsorption of the analytes to the beads is allowed for two minutes. The sample matrix is removed with a pipette after again applying the magnet. Beads are washed two times with each 250 µl of water. After the second washing step the beads are resuspended in 100 µl of methanol in water (90+10). After two minutes, the beads are captured magnetically and the supernatants are transferred into HPLC vials. The entire extraction procedure is performed at ambient temperature. LC-MS/MS analysis of the extract is performed as described previously (Vogeser M, Spohrer U, CLINICAL CHEMISTRY AND LABORATORY MEDICINE 44 (9): 1126-1130 SEP 2006) using a Waters Micromass Quattro Ultima Pt instrument.

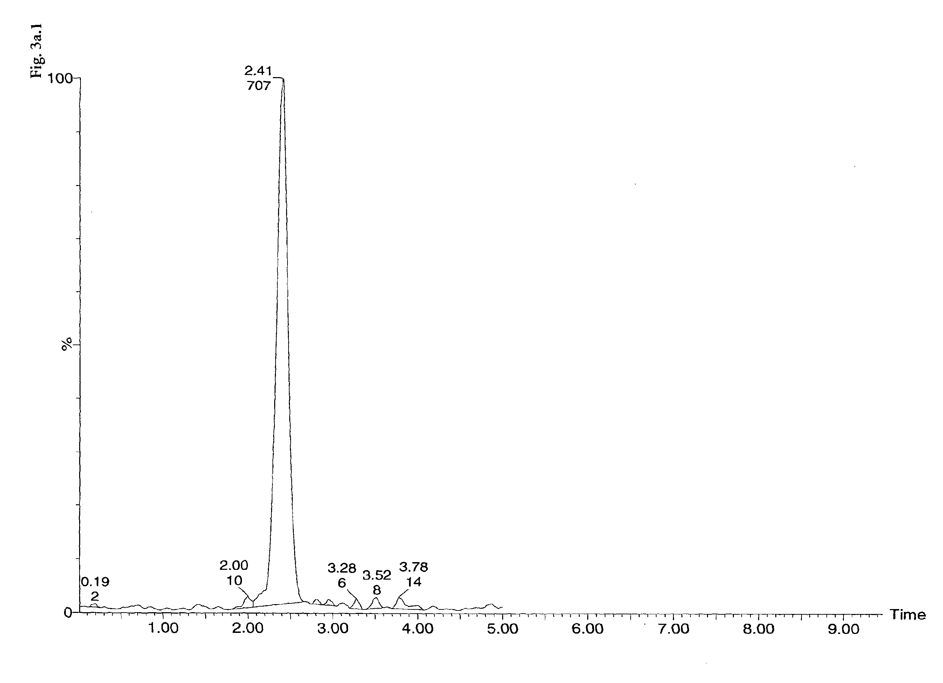

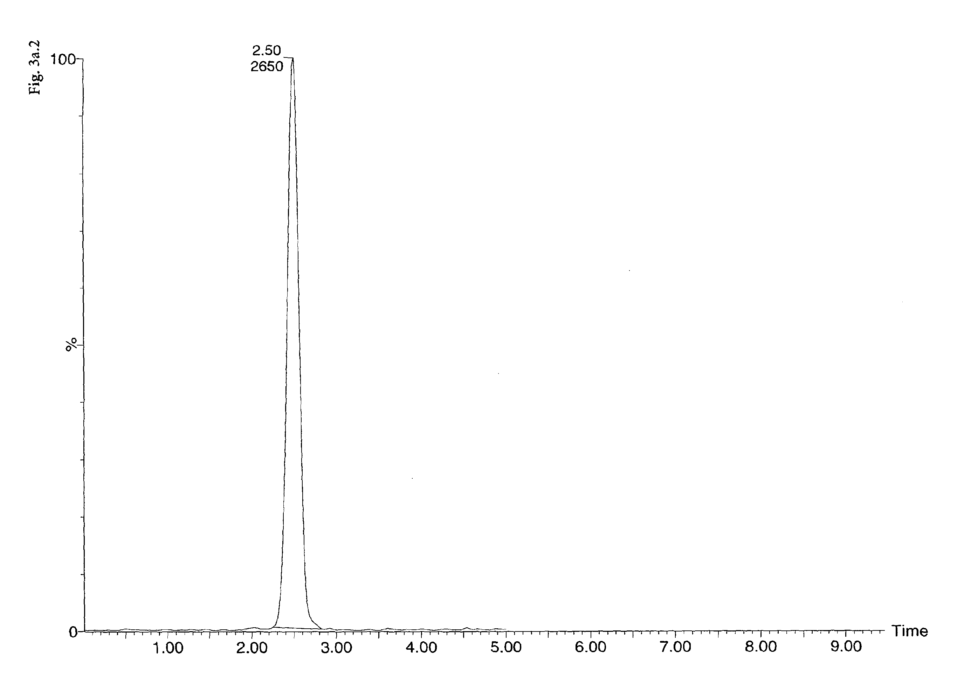

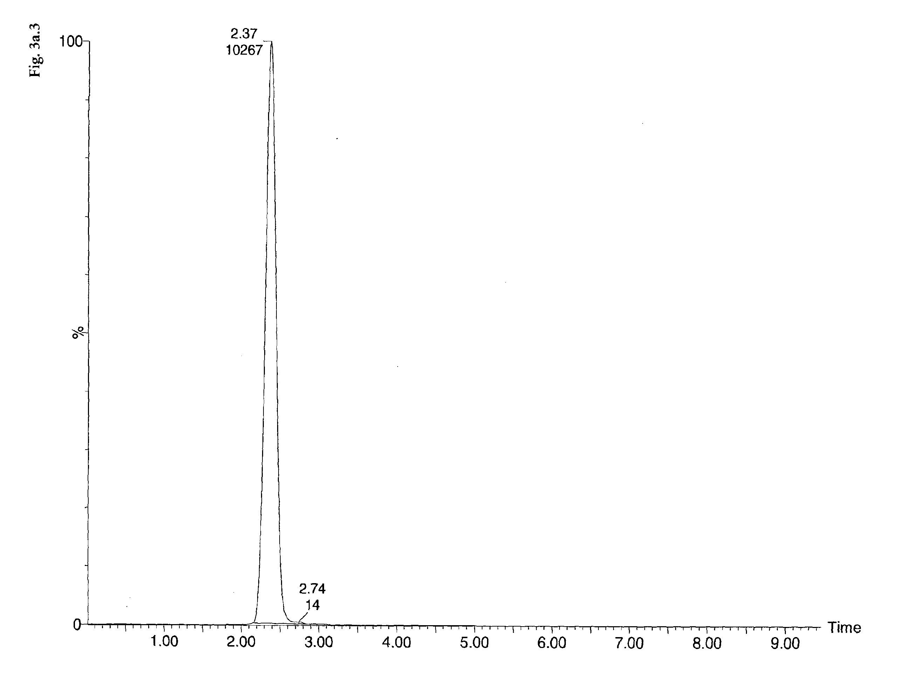

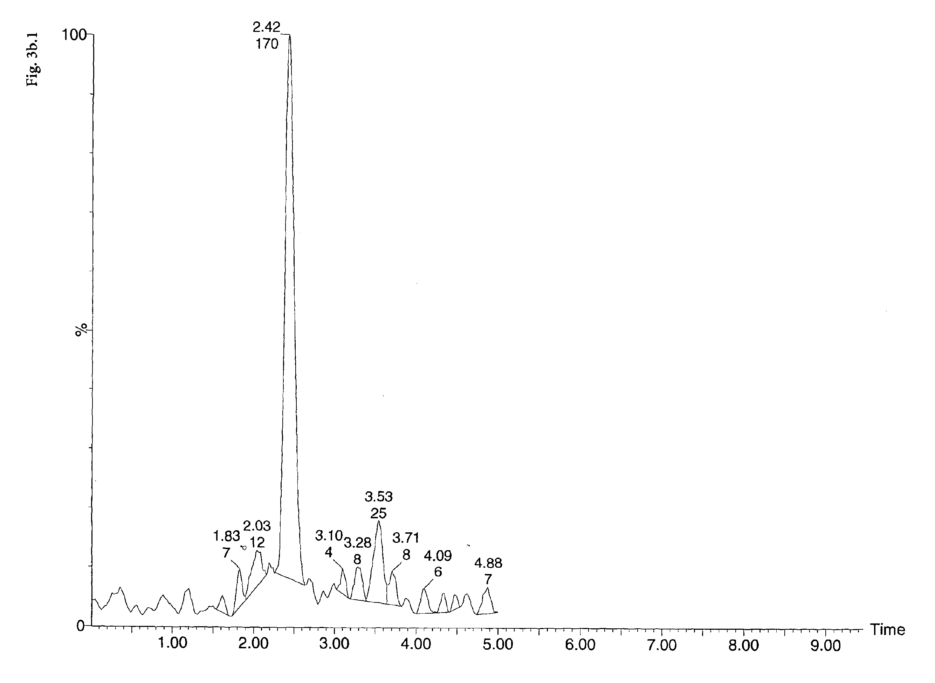

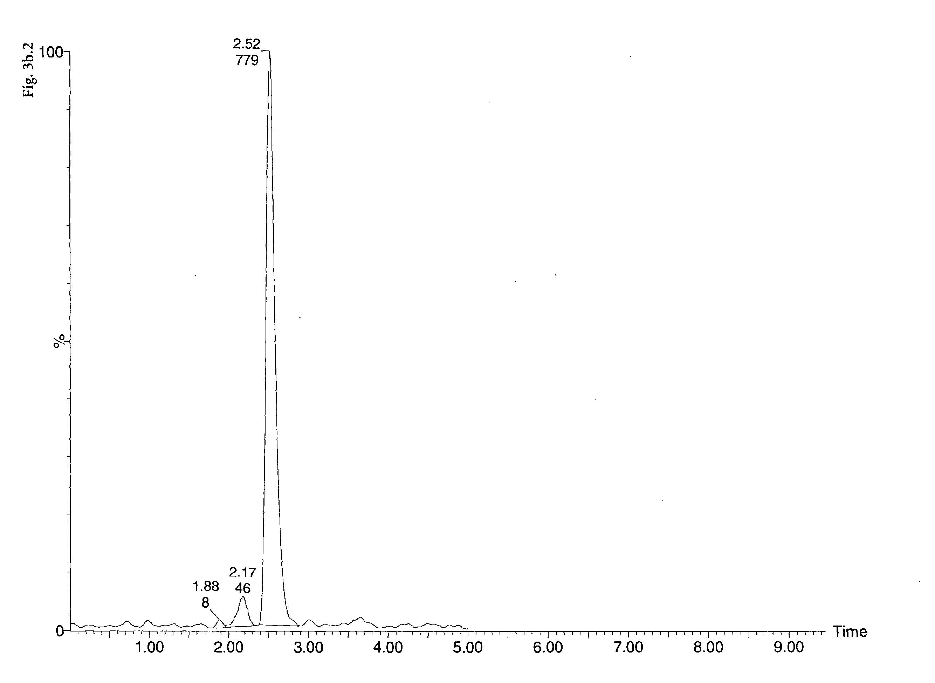

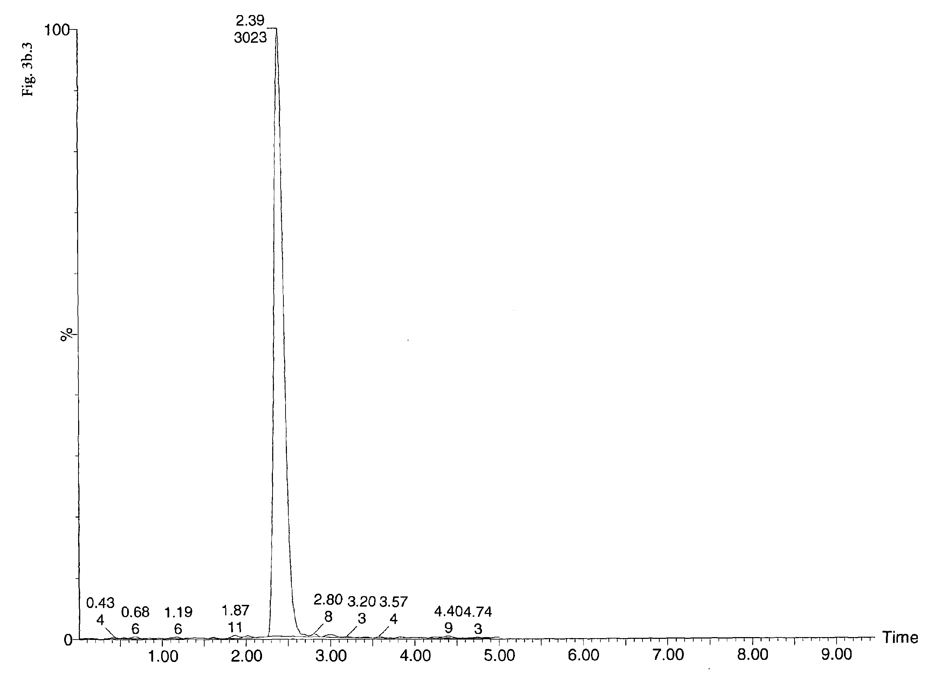

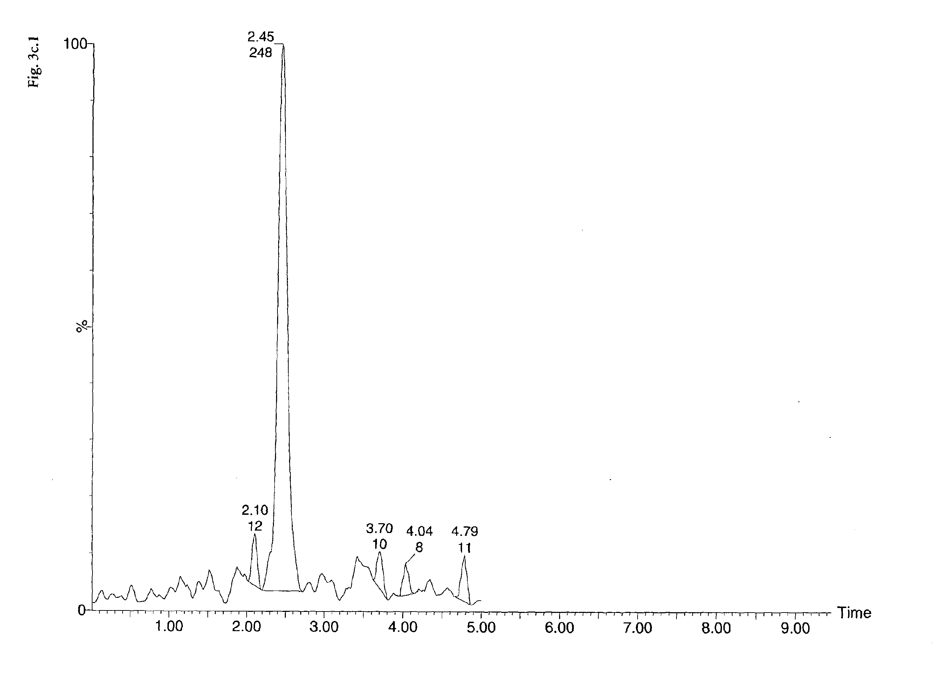

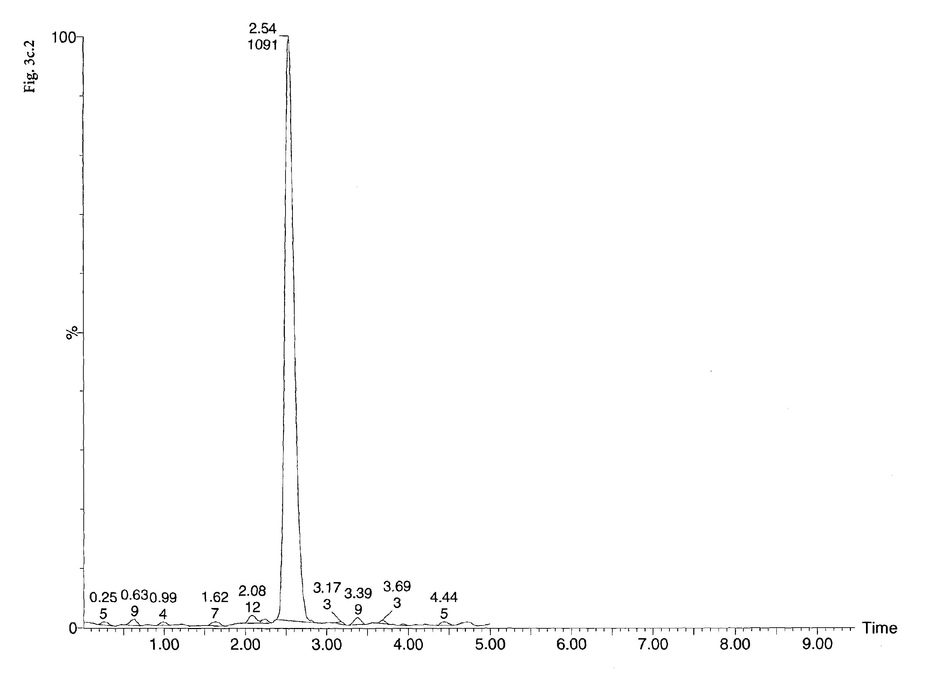

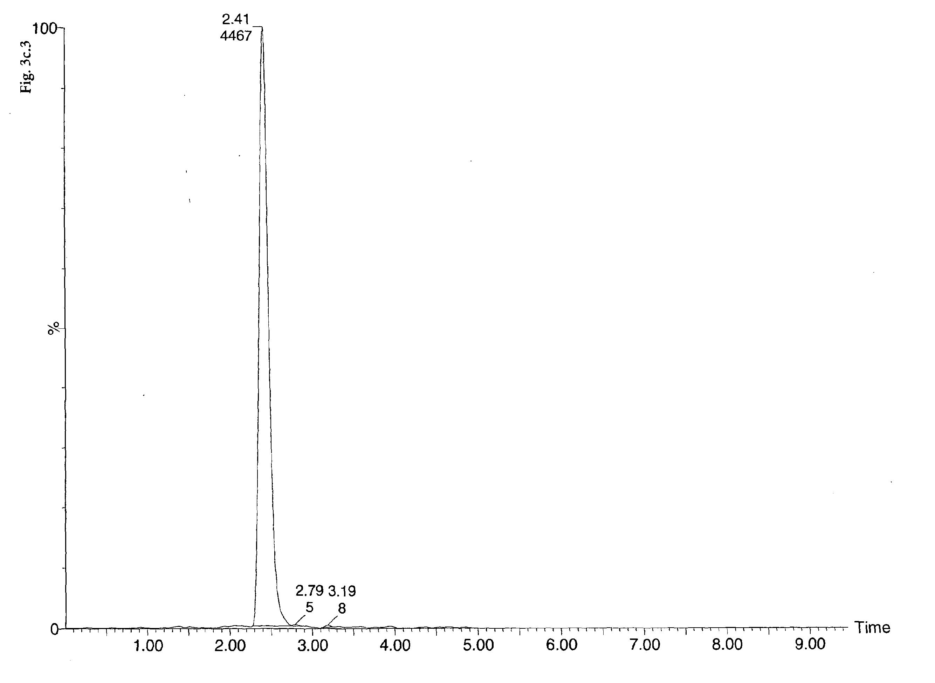

Typical chromatogramms are shown in Figure 3

### Example 5

### Analysis of a serum patient sample containing mycophenolic acid

For the demonstration of the invention patient serum samples from patients treated with mycomophetilphenolat drugs are used as analytes. Mycophenolic acid (MPA) as active metabolite of mycomophetilphenolat is used as analyte and determined by HPLC-UV.

In an exemplary case commercially available C18-functionalized magnetic particles (= beads) (Dynal^{®} Dynabeads^{®} RPC18 (Prod. No. 102.01), Invitrogen, Karlsruhe, Germany; 50 mg/ml, mean particle diameter 1 µm) are used. The beads have a surface modified with C18 alkyl groups. Such particles are so far specified for the preparation of serum samples for MS-based protein analyses.

Whole blood samples from human patients are extracted using magnetic particles. In general the standard handling protocol given by the manufacturer is followed. 50 µl of the resuspended particles are transferred into 1.5 ml standard polypropylene recation tubes placed in a magnetic particle separator (Dynal MPC-S Magnetic Particle Concentrator, Product No. 120.20, Invitrogen). A permanent magnet is introduced into the separator device, leading to immobilization of the beads on the wall of the tubes. The particle free liquid is removed with a pipette; then, the magnet is removed from the separator device.

For condtioning of the beads, 250 µl of 0.1% [volume by volume] trichloroacetic acid is dispensed into the tubes; the bead pellets are re-dissolved by multiple pipetting the liquid within the tubes. The magnet is applied again and trichloroacetic acid is removed. This conditioning step is repeated. The conditioned beads are resuspended in 50 µl of formic acid (0.1% [volume by volume]); 50 µl of the internal standard solution and 50 µl of whole blood sample are added. With a pipette the content of the tubes is aspirated and blown out several times for mixing. Adsorption of the analytes to the beads is allowed for two minutes. The sample matrix is removed with a pipette after again applying the magnet. Beads are washed two times with each 250 µl of water. After the second washing step the beads are resuspended in 100 µl of methanol in water (90+10). After two minutes, the beads are captured magnetically and the supernatants are transferred into HPLC vials. The entire extraction procedure is performed at ambient temperature. LC-MS/MS analysis of the extract is performed as described previously (Vogeser M, et al.) using a HPLC-UV instrument.

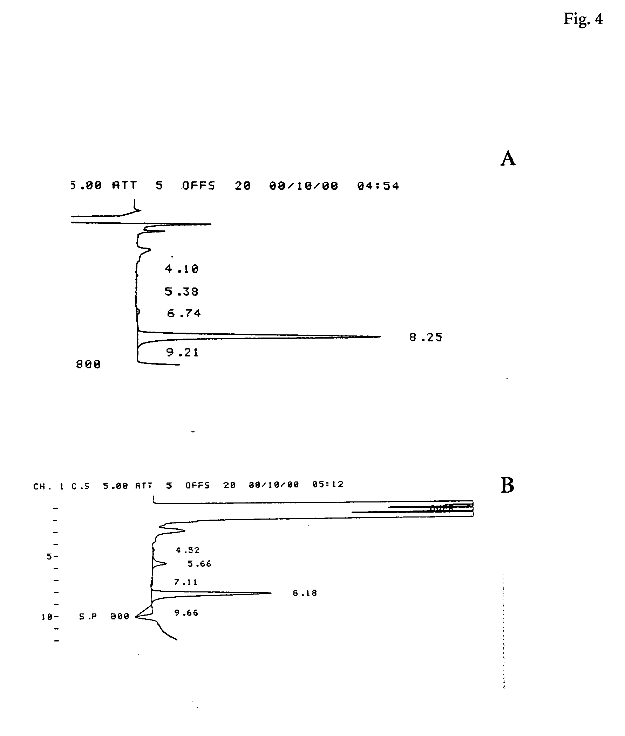

Typical chromatogramms are shown in Figure 4

### Example 6

### Preparation of hydrophobized magnetite nanoparticles

In general, preparation of ferrofluids was performed according to US 3,843,540 and Ramirez (Macromol. Chem. Phys. 2003, 204, 22-31).

14.6 g ferric chloride and 12 g of ferrous chloride tetra-hydrate are dissolved in 50 ml distilled water and 40 ml of ammonium hydroxide solution is added. After co-precipitation of magnetite nanoparticles, oleic acid (45% [weight by weight] with respect to the magnetite) is added and the supspension is heated to 70°C for 30 min. After that, the temperature is increased to 110°C in order to evaporate water and excess of ammonia. The sediment is colled to room temperature and is washed several times with distilled water.

### Example 7

### Preparation of water-based ferrofluid

In general, preparation of ferrofluids was performed according to US 3,843,540 and Ramirez (Macromol. Chem. Phys. 2003, 204, 22-31).

5 g of the magnetite powder from Example 6 (Preparation of hydrophobized magnetite nanoparticles) is added to 30 g of octane. This dispersion is poured into a solution consisting of 120 g water and SDS (12% [weight by volume] with respect to the dispersed phase consisting of octane, magnetite and oleic acid). The suspension is mixed for 1 h and sonicated with a UP200s (Dr. Hielscher GmbH) in an ice cooled bath. Octane is evaporated at 80°C and water is added from time to time to compensate the co-evaporated water.

### Example 8

### Preparation of polystyrene based seed latex particles

6 g SDS is dissolved in 475 ml water and stirred at 225 r.p.m. in a reaction vessel. The solution is degassed with nitrogen. 26 ml styrene is added and the resulting emulsion is stirred for 50 min at 80°C in an argon atmosphere. 25 ml of an aqueous sodium hydrogen carbonate solution (10% [weight by volume]) is added. The solutions is stirred for additional 10 min. The polymerization reaction is initiated by adding 2.5 ml of ammonium peroxo disulfate solution (326 mg in 2.5 ml degassed water). After 4 h at 80°C, 3 M hydrochloric acid is added to neutralize the pH. The suspension is filtered hot. The solid content is adjusted to 4% [weight by volume].

### Example 9

### Preparation of magnetic particles by seeded growth polymerization

### Step 1:

3 ml Toluene are dispersed with 152.4mg bisbenzoyl peroxide in 30 ml aqueous SDS solution (0.25% [weight by volume]. 1 ml of the seed latex particles from Example 8 (Preparation of polystyrene based seed latex particles) is added and the dispersion is stirred for 24 h.

### Step 2

2 ml of hexadecane, 10 ml of water and 30 mg of SDS are homogenized to an emulsion. To this emulsion, 125 ml of the ferrofluid from Example 7 (Preparation of water-based ferrofluid) is added and mixed with the dispersion from step 1 in a reactor.

### Step 3:

5.8 g Cyclohexanol, 3.75 g glycidylmethacrylate and 2.5 g ethylene glycoldimethacrylate are dispersed in 50ml aqueous SDS (0.25% [weight by volume]) and is stirred at room temperature for 3 hours. The resulting dispersion is added the dispersion of step 1 and stirred for additionl 24 h at room temperature A solution of 2 g PVA (Erkol W 25 / 140) in 50ml water is added and the reaction mixture is degassed for 20 min using nitrogen. The polymerization is done at 70°C for 24 h. The resulting precipitate is washed twice in water and methanol.

### Example 10

### Pore size specific hydrolysis and C8-surface modification

0.5 g of the particles from Example 9 (Preparation of magnetic particles by seeded growth polymerization) are treated for 48 h with 10 ml aqueous polystyrene sulfonic acid (1% [weight by volume]). The product is filtered and washed several times with water to neutralize the pH.

The particles are added to a solution of 14 mg potassium hydroxide in 3.5 g hot octanol. The reaction is done for 10 hours at 70°C. The reaction mixture is diluted with 20 ml dioxane and cooled to room temperature The particles are filtered and washed several times with water.

The product is redisperged in 10 ml 0.1 N sodium hydroxide solution and stirred for 15 h at room temperature to inactivate remaining epoxy functional groups. The filtered product is washed with water and redispersed in destilled water to a solid content of 2% [weight by volume].

## Claims

1. Use of functionalized magnetic particles with a hydrophobic surface for extracting a compound with low molecular weight from a complex liquid biological sample.

2. The use according to claim 1, **characterized in that** the molecular weight of the compound is between 50 Da and 1,000 Da.

3. The use according to any of the claims 1 or 2, **characterized in that** the sample is selected from the group consisting of serum, plasma, whole blood, and hemolyzed blood.

4. The use according to any of the claims 1 to 3, **characterized in that** the compound is a biologically active compound or a metabolite thereof.

5. The use according to any of the claims 1 to 4, **characterized in that** the compound is analyzed by way of mass spectrometry.

6. A method to purify a low molecular weight compound from a complex liquid biological sample, comprising the steps of
(a) contacting the sample with an amount of functionalized magnetic particles with a hydrophobic surface,
(b) incubating the sample and the particles, thereby adsorbing the compound to the hydrophobic surface,
(c) separating the particles by applying a magnetic field and removing the liquid,
(d) optionally washing the particles,
(e) eluting the compound from the particles.

7. The method according to claim 6, **characterized in that** the surface of the magnetic particles is functionalized with a chemical group selected from the group consisting of a C4-C30 alkyl group, a copolymer of 1-vinyl-pyrrolidon and a comdivinylbenzene, and a copolymer of styrene and divinylbenzene.

8. A method to assay a compound with low molecular weight in a liquid biological sample by way of mass spectrometry, comprising the steps of
(a) contacting the sample with an amount of functionalized magnetic particles with a hydrophobic surface,
(b) incubating the sample and the particles, thereby adsorbing the compound to the surface,
(c) separating the particles by applying a magnetic field and removing the liquid,
(d) optionally washing the particles,
(e) eluting the compound from the particles, and
(f) detecting the compound in the eluate by way of mass spectrometry.

9. The method according to claim 8, **characterized in that** the compound is detected by way of liquid chromatography and mass spectrometry.
